# EUROPEAN PATENT SPECIFICATION

(11) **EP 1 019 119 B1**
(45) Date of publication and mention of the grant of the patent: **16.02.2005**
(21) Application number: 98966956.9
(22) Date of filing: 23.09.1998
(51) Int. Cl.: A61M 25/06

(54) **INTRAVASCULAR CATHETER SYSTEM WITH CONVERTIBLE SHEATH EXTENSION FOR MAGNETIC RESONANCE IMAGING AND METHOD**
INTRAVASKULARES KATHETERSYSTEM MIT VERÄNDERLICHER HÜLLENEXTENSION FÜR DIE BILDERZEUGUNG MITTELS MAGNETISCHER RESONANZ SOWIE VERFAHREN
SYSTEME DE CATHETER INTRAVASCULAIRE A EXTENSION DE GAINE CONVERTIBLE POUR IMAGERIE PAR RESONANCE MAGNETIQUE ET PROCEDE

(30) Priority: 26.09.1997 NL 1007148
(43) Date of publication of application: 19.07.2000
(73) Proprietor: Cordis Corporation, Miami Lakes Florida 33014 (US); Hurtak, Wenzel Franz, 9301 TZ Roden (NL); Mous, Frans, 9203 HP Drachten (NL); Nap, Cornelis Philipus, 9345 AG Zevenhuizen (NL)
(72) Inventor: HURTAK, Wenzel, Franz, NL-9301 TZ Roden (NL); MOUS, Frans, NL-9203 HP Drachten (NL); NAP, Cornelis, Philipus, NL-9345 AG Zevenhuizen (NL)
(74) Representative: 't Jong, Bastiaan Jacobus
(86) International application number: PCT/US1998/019908
(87) International publication number: WO 1999/026682

(56) References cited:
- EP-A- 0 415 653
- EP-A- 0 416 467
- WO-A-96/24403
- US-A- 4 834 710
- US-A- 5 125 903
- US-A- 5 382 230
- US-A- 5 449 348

## Description

### BACKGROUND AND SUMMARY OF THE INVENTION

### 1. Technical Background:

The present invention relates generally to intravascular medical devices, and more particularly to an intravascular catheter system of the kind known from US-A 4 834 710.

### 2. Discussion:

The invention relates to a catheter introduction sheath. Such a catheter introduction sheath is arranged percutaneously in a blood vessel of a patient after which a guidewire, catheter etc. may be introduced through the introduction sheath into the vascular system of the patient. The usual catheter introduction sheath has been provided at its proximal end with a hemostatic valve in order to prevent loss of blood and through which the guidewire and catheter etc. are inserted.

With certain types of treatment, the site where a catheter introduction sheath has been introduced into the patient can not be reached or only with great difficulty, for instance when the patient has been received in the tunnel or room of a magnetic resonance imaging device. This device may surround the patient so closely that access to the introduction sheath is no longer possible. In order to introduce or change the guidewire or the catheters in that case, the patient will have to be pulled out of the magnetic resonance imaging device, so that the person carrying out the procedure can gain access to the introduction sheath. By pulling the patient repeatedly from the device and repositioning him inside it again, the treatment will last too long, which is uncomfortable to the patient and is in addition very expensive.

A good example of a catheter sheath introducer is described in the commonly assigned United States Patent number 4,874,378, issued to Hillstead on October 17, 1989, which is incorporated in this disclosure by reference.

As the body of the patient is of course opaque, physicians commonly use fluoroscopy or X-ray video cameras to track the position of intravascular devices within the body of a patient. In contrast to fluoroscopy, another method of visualizing the patient is magnetic resonance imaging, referred to as MRI. Other medical fields, such as neurology, often use procedures which are performed under magnetic resonance imaging instead of X-ray fluoroscopy.

The present invention provides an intravascular catheter system that includes a catheter introduction sheath, as well as a convertible sheath extension for use during magnetic resonance procedures. The catheter introduction sheath includes a tubular basic body with a hemostatic valve at its proximal end, and is adapted to allow the selective insertion of various intravascular devices, such as catheters and guidewires. The catheter sheath introducer is inserted percutaneously within the vascular system of a patient. The hemostatic valve resists leaking of blood, while automatically opening and closing to allow vascular devices of differing types to be inserted and removed. In addition, the convertible sheath extension has a selectively detachable coupling adapted to cooperate with the valve housing of the catheter sheath introducer. The convertible sheath extension thus extends the length of the catheter sheath introducer without interfering with the hemostatic valve, and can be coupled and removed from the catheter sheath introducer as desired. However, the convertible sheath extension does not increase the chance of blood coagulating in the system, since its lumen is bloodless due to the intermediate location of the hemostatic valve. In addition, the convertible sheath extension may be transparent, to allow an operator to see vascular devices inserted therein. The convertible sheath extension may have various preselected flexibilities.

As the hemostatic valve has been arranged directly behind the insertion section, the guiding section will remain free of blood, as a result of which the risk of thrombi due to blood clots will be avoided in this section of the introduction sheath. The insertion section with the hemostatic valve connected to it may be introduced into the patient without the guiding section. Due to the absent extra length of the guiding section, the insertion section can be manipulated properly and carefully.

After positioning the insertion section together with the hemostatic valve, the guiding section may be arranged, after which the treatment may be carried out.

Preferably the basic body is made in such a way that it is stiffly flexible, which means that it has a considerably greater bending stiffness than a catheter or a guidewire, but is not entirely stiff.

In another embodiment, the element inserted into the guiding section of the introduction sheath is clearly visible. Accordingly, it can be established properly when the distal end of this element has reached the hemostatic valve, and that resistance experienced on further introduction is caused by this valve.

These and various other objects, advantages and features of the invention will become apparent from the following description and claims, when considered in conjunction with the appended drawings.

### BRIEF DESCRIPTION OF THE DRAWINGS

Figure 1 is a perspective view of an intravascular catheter system for use with magnetic resonance imaging, arranged according to the present invention; and
Figure 2 is a partial longitudinal cross-section view of an intravascular catheter system for use with magnetic resonance imaging, arranged according to the present invention.

### DETAILED DESCRIPTION OF THE PREFERRED EMBODIMENT

The following description of the preferred embodiments of the present invention is merely illustrative in nature, and as such it does not limit in any way the present invention, its application, or uses. Numerous modifications may be made by those skilled in the art without departing from the true spirit and scope of the invention.

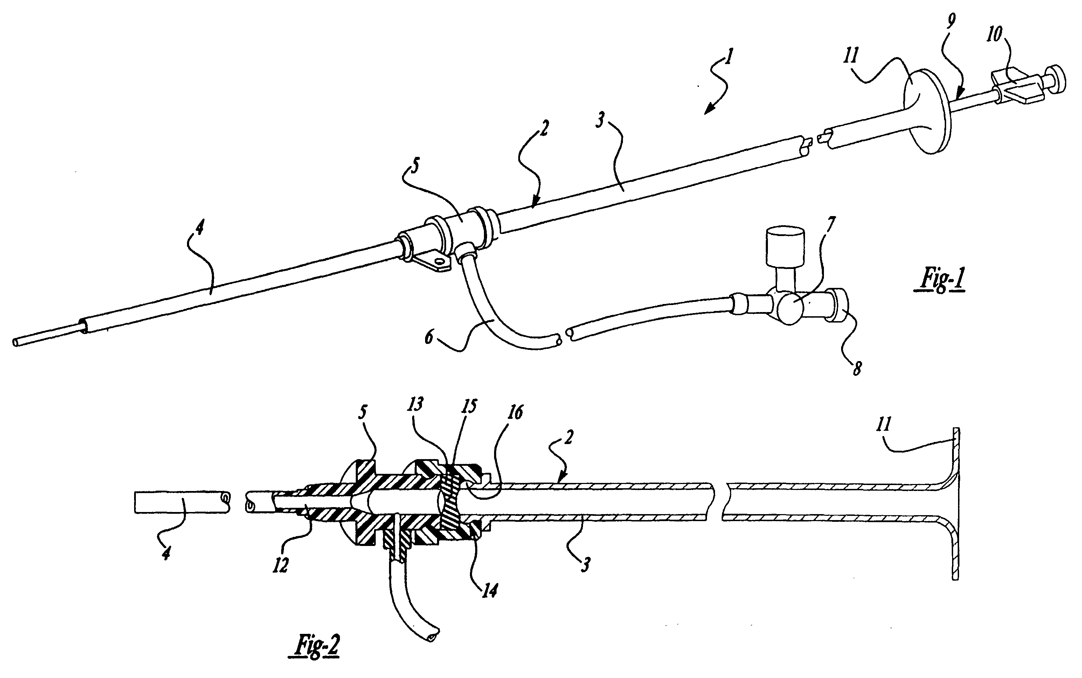

The introduction sheath 1 illustrated in Figure 1 includes a tubular basic body 2. The tubular basic body is in the example of this embodiment assembled from a convertible sheath extension 3, an insertion section 4 and arranged in between, a valve housing 5 comprising a hemostatic valve 13.

The insertion section 4 is introduced percutaneously into a blood vessel of a patient in a manner known as such. Via the lumen 12 of the basic body 2, for instance a catheter 9 may be introduced subsequently into the vascular system of the patient. The catheter with the connecting member 10 has only been given here by way of an example. Also for instance a guidewire, a guiding catheter etc. may be introduced into a patient in the usual manner via an introduction sheath.

After introducing the insertion section 4, the hemostatic valve housing 5 is usually situated close to the site where the insertion section 4 has been 35 introduced through the skin of the patient. This may for instance be in the inguinal area of the patient.

In case a magnetic resonance imaging procedure is to be carried out, and the room of the magnetic resonance imaging device which is to be used is so narrow that the inguinal area of the patient can no longer be reached when he is positioned inside the imaging device, the required supply and removal of the guidewire and/or catheters can be effected nonetheless via the convertible sheath extension 3, as the convertible sheath extension 3 forms a means of distant control for the insertion section 4.

The chosen length of the guiding section 4 is such that under the specific circumstances the proximal 10 end section of this convertible sheath extension 3 remains properly accessible so that elements such as a catheter 9 may be introduced into the patient.

At the proximal end of the convertible sheath extension 3 a supporting edge 11 has been formed, which provides a good support for the fingers of the person carrying out the procedure and which can be used for instance in order to generate the required counter pressure when pushing the catheter 9 through the hemostatic valve 13.

With the embodiment illustrated here, the lumen 12 has been provided with a branch pipe inside the hemostatic valve housing 5, to which a tube 6 has been connected which carries a tap 7 at its proximal end. Via the connection 8 of the tap 7 a fluid may be supplied to the lumen 12 and from there into the blood stream of the patient.

With the embodiment shown, the guiding section of the basic body 2 has been connected with the hemostatic valve housing 5 by means of a snap connector 14. This snap connector 14 is formed by a groove 15 extending around the lumen 12 into which a bulge 16, arranged at the distal end of the convertible sheath extension 3, can engage. By exerting an axial pressure to the convertible sheath extension 3, the latter may be clicked into the hemostatic valve housing 5 or removed from it.

After removing the convertible sheath extension 3 in this manner the hemostatic valve 13 remains active so that no unwanted loss of blood will occur.

As has been mentioned above, the guiding section 3 has been made of a transparent plastic material, so that the progress of the element inserted, like the catheter 9; can be monitored properly and that it can be established for instance when the distal end of the catheter inserted has to be pushed through the hemostatic valve 13.

The flexibility of the basic body 2 of the catheter introduction sheath 1 may vary along its length. The insertion section 4 may for instance be relatively flexible in order to prevent trauma and the convertible sheath extension 3 relatively stiff so that it can be handled easily. The stiffness of both the convertible sheath extension 3 and the insertion section 4 may vary along their lengths, depending on the required properties.

Furthermore, the lumen 12 may have been provided with a lubricating layer, so that the elements to be inserted, such as the catheter 9, can be passed through the introduction sheath 1 experiencing little friction.

It should be understood that an unlimited number of configurations for the present invention can be realized. The foregoing discussion describes merely exemplary embodiments illustrating the principles of the present invention, the scope of which is recited in the following claims.

## Claims

1. An intravascular medical catheter system for use in intravascular medical procedures, comprising:
a catheter sheath introducer having proximal and distal ends including a tubular basic body (2), and a hemostatic valve (13) disposed at the proximal end of the catheter sheath introducer, the hemostatic valve (13) resiliently opening upon the introduction of a medical device, and incorporating a first fitting (14),
a convertible sheath extension (3) having a second fitting (16) disposed at a distal end of the convertible sheath extension (3), and
a detachable coupling formed by the first (14) and second fittings (16), adapted to allow the catheter sheath introducer and the convertible sheath extension (3) to be selectively coupled and decoupled,
whereby said hemostatic valve (13) resists blood from escaping whether or not the convertible sheath extension (3) is coupled, **characterized by** the convertible sheath extension (3) having a fixed length and including a tubular shaft.

2. The catheter system as set fourth in claim 1, wherein the basic body (2) is stiffly flexible.

3. The catheter system as set fourth in claim 1, wherein at least the convertible sheath extension (3) has been made of a transparent plastic material.

4. The catheter system as claimed in claim 1, wherein the convertible sheath extension (3) has been connected to the catheter sheath introducer by a snap connector (14, 16).

5. The catheter system as claimed in claim 1, wherein the inside of the tubular shaft has been provided with a coating for reducing friction.

## Patentansprüche

1. Intravaskuläres, medizinisches Katheter-System zur Verwendung in intravaskulären, medizinischen Verfahren, aufweisend:
einen Katheterhülse-Einführer mit einem Proximal- und einem Distal-Ende, welcher einen rohrförmigen Basiskörper (2) und ein hämostatische Ventil (13) aufweist, welches am Proximal-Ende des Katheterhülse-Einführers angeordnet ist, wobei das hämostatische Ventil (13) sich nach dem Einführen einer medizinischen Vorrichtung federnd öffnet und einen ersten Anschlussteil (14) inkorporiert,
eine konvertible Hülsen-Verlängerung (3) mit einem zweiten Anschlussteil (16), welches am Distal-Ende der konvertiblen Hülsen-Verlängerung (3) angeordnet ist, und
eine von dem ersten (14) und dem zweiten Anschlussteil (16) gebildete lösbare Kupplung, welche eingerichtet ist, dass der Katheterhülsen-Einführer und die konvertible Hülsen-Verlängerung (3) wahlweise ankuppelbar und auskuppelbar sind,
wobei das hämostatische Ventil (13) sich widersetzt, dass Blut austritt, ob die konvertible Hülsen-Verlängerung (3) angekuppelt ist oder nicht, **dadurch gekennzeichnet, dass** die konvertible Hülsen-Verlängerung (3) eine feste Länge hat und einen rohrförmigen Schaft aufweist.

2. Katheter-System gemäß Anspruch 1, wobei der Basiskörper (2) steif-flexibel ist.

3. Katheter-System gemäß Anspruch 1, wobei zumindest die konvertible Hülsen-Verlängerung (3) aus einem transparenten Kunststoffmaterial hergestellt ist.

4. Katheter-System gemäß Anspruch 1, wobei die konvertible Hülsen-Verlängerung (3) mit dem Katheterhülsen-Einführer durch eine Einrast-Verbindung (14, 16) verbunden ist.

5. Katheter-System gemäß Anspruch 1, wobei die Innenseite des rohrförmigen Schafts mit einem Überzug versehen ist zum Reduzieren der Reibung.

## Revendications

1. Système de cathéter médical intravasculaire destiné à être utilisé dans des actes médicaux intravasculaires, comprenant :
un introducteur de cathéter à gaine ayant des extrémités proximale et distale comprenant un corps de base tubulaire (2), et une valve hémostatique (13) disposée au niveau de l'extrémité proximale de l'introducteur de cathéter à gaine, la valve hémostatique (13) s'ouvrant de manière élastique au moment de l'introduction d'un dispositif médical, et comprenant un premier raccord (14),
une extension de gaine convertible (3) comportant un second raccord (16) disposé au niveau d'une extrémité distale de l'extension de gaine convertible (3), et
un couplage détachable formé par le premier (14) et le second (16) raccords, adapté pour permettre à l'introducteur de cathéter à gaine et à l'extension de gaine convertible (3) d'être couplés et découplés de manière sélective,
moyennant quoi ladite valve hémostatique (13) empêche le sang de fuir, que l'extension de gaine convertible (3) soit couplée ou non,
**caractérisé en ce que** l'extension de gaine convertible (3) a une longueur fixe et comprend un arbre tubulaire.

2. Système de cathéter selon la revendication 1, dans lequel le corps de base (2) est fermement flexible.

3. Système de cathéter selon la revendication 1, dans lequel au moins l'extension de gaine convertible (3) est réalisée dans une matière plastique transparente.

4. Système de cathéter selon la revendication 1, dans lequel l'extension de gaine convertible (3) est reliée à l'introducteur de cathéter à gaine par un raccord d'accrochage (14, 16).

5. Système de cathéter selon la revendication 1, dans lequel l'intérieur de l'arbre tubulaire est doté d'un revêtement destiné à réduire le frottement.
